(19) Europäisches Patentamt
European Patent Office
Office européen des brevets

(11) EP 2 254 560 B1

(12) EUROPEAN PATENT SPECIFICATION

(45) Date of publication and mention
of the grant of the patent:
**22.01.2014 Bulletin 2014/04**

(51) Int Cl.:
***A61K 9/51*** (2006.01)

(21) Application number: **09705877.0**

(22) Date of filing: **29.01.2009**

(86) International application number:
**PCT/EP2009/051021**

(87) International publication number:
**WO 2009/095448 (06.08.2009 Gazette 2009/32)**

(54) **PREPARATION OF NANOPARTICLES BY USING A VIBRATING NOZZLE DEVICE**

ZUBEREITUNG VON NANOTEILCHEN MIT EINER VIBRIERENDEN DÜSENVORRICHTUNG

PRÉPARATION DE NANOPARTICULES À L'AIDE D'UN DISPOSITIF À BUSE VIBRANTE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **30.01.2008 EP 08150853**

(43) Date of publication of application:
**01.12.2010 Bulletin 2010/48**

(73) Proprietor: **LEK Pharmaceuticals d.d.**
**1526 Ljubljana (SI)**

(72) Inventors:
• **AHLIN GRABNAR, Pegi**
**1000 Ljubljana (SI)**
• **ZVONAR, Alenka**
**1000Ljubljana (SI)**
• **KRISTL, Julijana**
**1000 Ljubljana (SI)**

• **JURKOVIC, Polona**
**1526 Ljubljana (SI)**
• **KERC, Janez**
**1526 Ljubljana (SI)**

(74) Representative: **Prüfer & Partner GbR**
**European Patent Attorneys**
**Sohnckestrasse 12**
**81479 München (DE)**

(56) References cited:
**EP-A- 0 235 603        WO-A-2005/072709**
**DE-A1- 10 024 154      US-A1- 2002 054 912**

• **SCHNEIDER ET AL: "Source of Uniform-Sized Liquid Droplets" THE REVIEW OF SCIENTIFIC INSTRUMENTS, vol. 35, no. 10, 1964, pages 1349-1350, XP002526716**

## Description

The production of small particles is important in many technical fields including but not limited to pharmaceutics, nutraceutics, food processing, paints and copying technologies. In general, smaller particles sizes may lead to the development of new products as well as to more effective products. New techniques for generating particles with decreased particle size are necessary for future development in many scientific and industrial fields.

The present invention relates to a method for a simple, reproducible and economical manufacturing of polymer nanoparticles. In particular, the present invention relates to a method for preparing nanoparticles with a nanoprecipitation method using a vibrating nozzle device. This automated technique presents some advantages such as high reproducibility, the possibility to scale up the production and the possibility for producing sterile nanoparticles.

The oral delivery of hydrophobic drugs presents a major challenge because of the low water solubility, and consequently low bioavailability, of such compounds. An important fraction of the dose is thus eliminated from the gastrointestinal tract before being absorbed. These problems could be reduced through association of drugs with polymeric nanoparticulate systems that have the ability to both control the release of the loaded drug and protect against its degradation. Moreover, the small particle size allows them to penetrate the mucus layer and thus bind to the underlying epithelium and/or adhere directly to the mucus network. Nanoparticles may therefore enhance the drug absorption rate by reducing the diffusion barrier between the pharmaceutical dosage form and the site of action or absorption, and by prolonging the residence time of the drug in the gut.

Ingredients which can be entrapped in the nanoparticles or adsorbed at the surface of the nanoparticles include any ingredient which it may be desired to administer to the human body for any purpose, including therapeutic, diagnostic, cosmetic and prophylactic agents. The additional ingredients may be ingredients for veterinary and agricultural use, food products or additives, colorants or any other ingredients.

At present, various techniques are known for preparing aqueous nanoparticle dispersions. These techniques include emulsification-evaporation, nanoprecipitation, salting-out and emulsification-diffusion.

The nanoprecipitation technique is a known technique for the preparation of nanoparticles. In this method, a polymer, a drug and, optionally, a lipophilic stabilizer (e.g. phospholipids) are dissolved in a semipolar water-miscible solvent, such as acetone or ethanol. This solution is poured or injected into an aqueous solution containing a stabilizer (e.g. polyvinyl alcohol (PVAL) or poloxamer 188) under magnetic stirring. During this procedure, nanoparticles are formed instantaneously by the rapid diffusion of the solvent, which is then eliminated from the suspension under reduced pressure.

In order to obtain a particle size in the nanometric range a good distribution of organic phase into aqueous phase is crucial. In laboratory scale this can be achieved by agitation of the aqueous phase during the addition of an organic polymer solution. However, with enlargement of the volume this method becomes ineffective. Therefore, hitherto the nanoparticle production by nanoprecipitation is limited to small quantities and requires a lot of time. Accordingly, until the present invention a scale up of the nanoprecipitation method was difficult.

US Patent No. 5,118,528 discloses the original method of nanoprecipitation. The method according to this document comprises: (1) the preparation of a liquid phase consisting essentially of a solution of the substance in a solvent or in a mixture of solvents to which may be added one or more surfactants, (2) the preparation of a second liquid phase consisting essentially of a non-solvent or a mixture of non-solvents for the substance and to which may be added one or more surfactants, the non-solvent or the mixture of non-solvents for the substance being miscible in all proportions with the solvent or the mixture of solvents for the substance, (3) the addition of one of the liquid phases prepared in (1) or (2) to the other with moderate stirring so as to produce a colloidal suspension of nanoparticles of the substance, and (4) is desired, the removal of all or part of the solvent or the mixture of solvents for the substance and of the non-solvent or the mixture of non-solvents for the substance so as to produce a colloidal suspension of nanoparticles of the desired concentration or to produce a powder of nanoparticles.

EP-A-1 728 814 discloses a method for forming particles using a porous membrane. According to this document, a solvent with the dissolved polymer is pumped from a solvent container and a flocculating agent is pumped from a flocculating agent container in two circulations and in two volumes divided by the porous membrane of a membrane reactor. The precipitated nanoparticles with size of 60 nm are filtered in a filtration step.

WO 2005/072709 A2 discloses a drug delivery system comprising particles of water insoluble or poorly soluble drugs in a nanosized or microsized form dispersed in a polymeric bead. The beads consisting of drug nanoparticles or microparticles are essentially free of water insoluble polymer and are micrometer sized or larger. The beads production process comprises formation of nanoemulsion or microemulsion, which is subsequently mixed with water-soluble bead-forming polymers and introduced into an Inotech encapsulator and jetted into crosslinking solution. Beads of micrometer size or larger are formed by solidifying drops either by contact with a crosslinking agent or by temperature induced solidification. After evaporating the volatile organic solvent and the water, dried polymeric beads containing nanodispersed or microdispersed poorly water soluble drug are obtained.

EP-A-0 235 603 discloses a method for forming uniform liquid droplets having a particle size of not more than 250 $\mu$m which comprises jetting a liquid having a viscosity of 50 to 2000 cP through at least one orifice having a diameter of 20 to 100 $\mu$m, while directly vibrating said liquid at a frequency of 300 to 40000 Hz with a vibrating rod. The frequency capable of giving the uniform liquid droplets is decided depending on the orifice diameter, the flow rate, the viscosity and surface tension of the liquid or the amplitude of the vibrating rod, but does not depend on the composition of the liquid. The method is applicable, for instance, to a solution of a natural or synthetic high polymer, and when liquid droplets formed from such a solution are solidified, spherical solid particles having a uniform particle size are obtained.

US 2004/0022939 A1 and US Patent No. 6,669,961, respectively, disclose a method of forming microparticles, which comprises accelerating a stream comprising a liquid and vibrating the stream, to form particles. The particle may have a diameter that is smaller than the diameter of the nozzle used to form the stream, allowing for the formation of micro- and nanosized particle. According to this method, the addition of an organic phase to the aqueous phase should be controlled and constant, by mild stirring, to assure a uniform distribution and diffusion.

Fessi et al., 1989 H. Fessi, F. Puisieux, J.P. Devissaguet, N. Ammoury and S. Benita, Nanocapsule formation by interfacial polymer deposition following solvent displacement, Int. J. Pharm. 55 (1989), pp. 25-28; describes a technological procedure for the production of nanocapsules.

Furthermore, there are several publications directed to methods for producing micro- and nanoparticles:

Prakobvaitayakit and Nimmannit (AAPS PharmSciTech, 4(4), 1-9 (2003)) used a constant flow rate of 0.3 ml/min with mechanical stirring of 750 rpm. In the work of Govender et al. (Journal of Controlled Release, 57, 171-185 (1999)) a dropwise organic phase addition has been reported. The stirring was done by a magnetic stirrer. The same procedure was followed by Saxena et al. (Int. J. Pharm, 278 (2), 293-301 (2004)). Csaba et al. (Journal of Biomaterials Science, Polymer edition, 15 (9), 1137-1151 (2004); Biomacromolecules, 6, 271-278 (2005)) used vortex agitation for mixing both phases getting a fast organic phase dispersion and further moderate magnetic stirring. Other works using fast organic phase dispersion are the publications of Ameller et al. (European Journal of Pharmaceutical Sciences, 21, 361-370 (2004); Pharmaceutical Research, 20(7), 1063-1070 (2003)).

The present invention provides a method for preparing nanoparticles according to claim 1, while preferred embodiments are set forth in dependent claims and will be further described below

The term "nanoparticles" (NP) as used in the present invention is directed to particles composed of polymer matrix and a drug having a diameter < 1000 nm. The nanoparticles according to the present invention are nanospheres.

The present method for the preparation of nanoparticles with a nanoprecipitation method using vibrating nozzle device (e.g. Encapsulator Inotech IE-50R, Inotech, Swiss) is automated technique and presents some advantages such as high reproducibility and the possibilities of scale up and preparation of sterile nanoparticles. According to the present invention the preparation of the nanoparticles may be continuous or non continuous.

The present invention is based on the idea that passing or pumping an organic phase through a nozzle and splitting this jet in very small droplets enables a better distribution of organic phase in an aqueous phase in comparison with injecting, leading to the formation of very small and homogeneous nanoparticles.

In the following, preferred embodiments of the invention are described.

In a method according to the present invention, nanoparticles may be prepared in the following way:

A polymer and, optionally, at least one additional ingredient, preferably a drug, is/are dissolved in an water miscible organic solvent (organic phase; e.g. acetone), passed, preferably pumped, through a vibrating nozzle and are dropped through an electrical field into an aqueous solution, which preferably comprises a stabilizer (e.g. an aqueous solution of polyvinyl alcohol), which aqueous solution is stirred (e.g. magnetically). A colloidal dispersion comprising nanoparticles is formed instantaneously by the rapid diffusion of the solvent, which may be then eliminated from the suspension by evaporating under atmospheric pressure at room temperature or under reduced pressure.

Biocompatible and biodegradable polylactides/glycolides (PLA/PLGA) have received high attention over the last thirty years in the biomedical field as sutures, implants, colloidal drug delivery systems, and more recently also in tissue repairing and engineering and anti-cancer drug delivery.

Accordingly, a matrix polymer which may be used in the present invention is preferably selected from biodegradable polymers, preferably hydrophobic polymers such as polylactide (PLA), copolymers of lactide and glycolide (PLGA), poly($\varepsilon$-caprolactone) (PCL).

However, depending on the intended use of the nanoparticles, it is also possible to use non-biodegradable polymers such as copolymers of acrylic and methacrylic acid esters, cellulose acetate phthalate (CAP), cellulose acetate trimellitate (CAT), and ethylene vinyl acetate copolymer (EVAC) or mixtures thereof.

The optimal initial drug : polymer ratio that could be used to produce nanoparticles with good entrapment efficiency depends on the properties of the drug to be incorporated and on the properties of matrix polymer, as well as composition of organic and aqueous phase. Depending on desirable entrapment efficiency and drug loading the initial drug : polymer ratios may be from 1:100 to 1:1, preferably between 1:20 and 1:5.

It is preferred that the polymer is present in the organic solvent (organic phase) in a concentration range of 0.1 to 10 % w/w.

Water-miscible organic solvents which may be used in the present invention are preferably selected on the basis of their volatility and low toxicity, in particular when a pharmaceutical application for the resulting aqueous colloidal dispersion is considered. The solvent or the mixture of solvents used is a liquid capable of dissolving the polymer and the drug. Moreover, the solvent is preferably miscible with the aqueous phase, optionally containing a stabilizer and sufficiently volatile in order to be removable after the product preparation. The solvent may preferably be chosen from among a lower alcohol (methanol, ethanol, isopropanol, etc.), a lower ketone (acetone, methyl-ethyl-ketone, 2-butanone, etc.), esters (ethyl acetate, methyl acetate, isopropyl acetate) or other common solvents such as acetonitrile, etc. Particularly preferred are acetone and ethanol because of their widely recognized low toxicity, good solubilizing properties and low boiling points.

The viscosity of organic phase comprising the polymer and optionally a further substance, is preferably in the range of 0.3 to 1 cP, and the surface tension is preferably in the range of 20 to 25 mN/m.

The method for the preparation of the nanoparticles is preferably conducted at room temperature (i.e. 20 to 25 °C) and a relative humidity of 40 to 60 %.

As described above, stabilizers or stabilizing agents of the hydrophilic phase (nonsolvent, aqueous solution) are preferably used in the present invention. These stabilizing agents are not specifically restricted as long as they are able to stabilize the hydrophilic phase. Stabilizing agents, which are particularly preferred for stabilizing the final dispersion are poly (vinyl alcohol) (PVAL) and poloxamers or mixtures thereof, because of their good water solubility, suitability for ingestion, lyoprotective properties and compatibility with the system. The stabilizing agent may be contained in the aqueous phase preferably in an amount of not more than 1 % w/w, and more preferably in an amount of not more than 0.1 % w/w, on the basis of the initial aqueous solution.

Stabilisers or stabilizing agents of the lipophilic phase (organic solvent) may be optionally used in the present invention. These stabilizing agents are not specifically restricted as long as they are able to stabilize the lipophilic phase. Suitable stabilisers of the lipophilic phase preferably include phospholipids, sorbitan esters (such as Span 60 - sorbitan monostearate), glyceryl monoesters (such as glyceryl monostearate), and nonyl phenol ethoxylates or any other stabiliser which is soluble in the organic solvent. The concentration of the lipophilic stabilizer is preferably 1 % (w/w) on the basis of the organic solvent.

The resulting colloidal suspension of nanoparticles may comprise 0.05 to 10 % w/w of at least one surfactant.

The delivery system prepared according to the method of the invention are particularly well suited for use with active compounds such as pharmacologically active proteins, peptides, vaccines, and the like, as well as with other small pharmacologically active molecules and contrast agents.

Specific active compounds (drugs) include celecoxib, budesonide, paclitaxel, camptothecin, 9-nitrocamptothecin, cisplatin, carboplatin, ciprofloxacin, doxorubicin, rolipram, simvastatin, methotrexate, indomethacin, probiprofen, ketoprofen, iroxicam, diclofenac, cyclosporine, etraconazole, rapamycin, nocodazole, colchicine, ketoconazole, tetracycline, minocycline, doxycycline, ofloxacin, octreotide, testosterone, progesterone, estradiol and estrogen.

The present invention provides a method for preparing nanoparticles, comprising the steps of:

dissolving a polymer and, optionally, at least one additional ingredient in an organic solvent,

passing the solution through a vibrating nozzle, and

dropping the solution through an electrical field into an aqueous solution, which is stirred,

wherein the vibrating nozzle has a frequency of vibration in the range from 50 to 7000 Hz, and wherein the electric field is built up between said vibrating nozzle and an electrode and a voltage in the range from 800 to 1800 V is applied, such that nanoparticles are formed by the rapid diffusion of the solvent.

Preferably the polymer is a hydrophobic polymer.

In the method of the present invention, preferably an encapsulator device is used. The core of the encapsulator nozzle may be a precisely drilled sapphire disc. Custom nozzles are available in aperture sizes of 50 to 1000 $\mu$m. Preferably, for the preparation of nanoparticles a 500 $\mu$m nozzle is used.

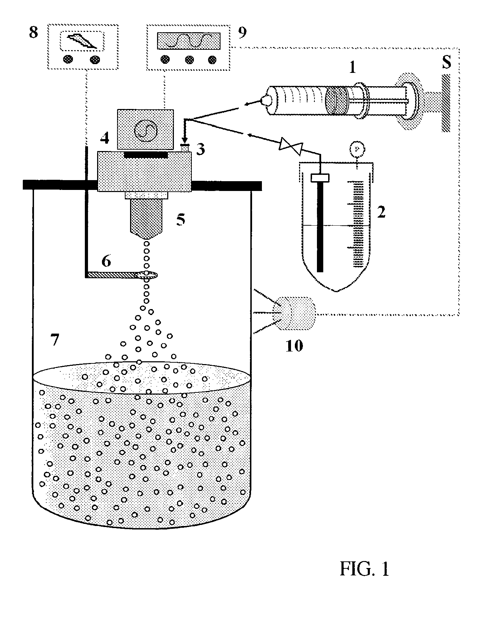

An example of a device, which may be preferable used in the method according to the invention, is shown in Fig. 1, wherein the numbers have the following meaning:

1     Syringe
2     Product delivery bottle
3     Pulsation chamber
4     Vibration system

5 Nozzle
6 Electrode
7 Reaction vessel
8 Electrostatic charge generator
9 Frequency generator
10 Stroboscope
P pressure control system
S syringe pump

The technology of vibrating nozzle device is based on the principle that a laminar jet is broken into equal sized droplets by a superimposed vibration. The vibrator is comprised of a disc magnet and an electrical coil through which alternating current flows and a part thereof is directly associated with the pulsation chamber. When alternating current is passed through the coil, it is alternately magnetised positively and negatively. The magnetic waves interact with the subjacent magnet attached to the deformable membrane and cause it to vibrate. In pulsation chamber the vibrations from the membrane are transmitted almost without resistance to the liquid jet which is then urged through the nozzle. Shortly after issuing from the nozzle the liquid jet breaks up into droplets of equal size, according to the frequency of the superimposed vibration.

Nanoparticles are produced in the frequency range between 50 and 7000 Hz, preferably at 450 and 550 Hz.

A feature of the Encapsulator which is preferably used in this is the ability to charge the surface of the droplets. When the droplets pass through an electrical field, which is built up between the electrode and the nozzle, a charge flux occurs in a direction of the nozzle so that the separated drops obtain an electrostatic charge. That similar charge causes mutual repulsion of the droplets. This prevents the droplets from hitting each other in flight, and from hitting each other as they enter the aqueous phase. Due to electrostatic repulsion the single-strand chain of droplets is expanded to form a cone, therefore the droplets are spread over the whole surface area of the aqueous phase. Consequently the coagulation of droplets is minimised, which increases the possibility of production of equally sized nanoparticles.

In a preferred embodiment of the present invention the method is conducted by using the following conditions:

The amplitude of the nozzle vibration may be between 1 and 7. For good nanoparticles production values between 1 and 4 are preferred.

The preferred flow rate for organic solvent through the device and the nozzle is from 8 to 10 ml/min.

The preferred applied voltage is in the range from 800 to 1000 V.

As mentioned above, the method for the preparation of nanoparticles according to the present invention has advantages such as automatization, high reproducibility, the possibility of scale up and the possibility of production of sterile nanoparticles. The preparation of the nanoparticles may be continuous or non continuous.

Nanoparticles prepared by the method of the present invention may be used as a powder or granules for oral suspension or may be packed in a suitable pharmaceutical formulation such as gelatine capsules or solid tablets.

**Examples**

The present invention is illustrated but in no way limited by the following examples, wherein the following materials, conditions and measuring methods were used:

*Freeze drying of nanoparticle dispersions*

Celecoxib loaded PLA/PLGA nanoparticle dispersions were freeze-dried. Samples were frozen at - 70 °C and freeze dried at 0.01 bar at room temperature.

*Particle size and zeta potential analysis*

The mean particle size and polydispersity index were estimated by a photon correlation spectroscopy (PCS) using a Zetasizer 3000 (Malvern, UK). The analysis was performed at a scattering angle of 90 ° and at a temperature of 25 °C. Before measuring samples were diluted with dust-free water to give the recommended scattering intensity. The diameter was calculated from the autocorrelation function of the intensity of light scattered from particles, assuming a spherical form for the particles. The polydispersity index (PI) is a measure of dispersion homogeneity and ranges from 0 to 1. Values close to 0 indicate a homogeneous dispersion while those greater than 0.3 indicate high heterogeneity.

The particle charge was quantified as zeta potential by laser Doppler anemometry using a Zetasizer 3000. The samples were diluted with distilled water adjusted to a conductivity of $50\mu$ $S/cm^2$ with a solution of 0.9 % NaCl. The zeta potentials were calculated by the Helmholtz- Smoluchowski equation.

*Determination of drug content in the nanoparticles*

The amount of celecoxib encapsulated per unit weight of NP was determined dissolving a weight amount of freeze dried NP in acetonitrile : water (1 : 1, v/v) mixture, filtered through 0.45 $\mu$m siringe filter in order to remove the precipitated PVA and than measuring the amount of the drug by an HPLC method using a Hypersil ODS C-18 column (5 $\mu$m, 300 x 3.9 mm; BIA Separations d.o.o. Ljubljana, Slovenia). The mobile phase was a mixture of acetonitrile, phosphate buffer (pH 4.0, 0.01 M) and methanol at a ratio of 45:45:10 (v/v). The detection wavelength was 238 nm and the flow rate 1.4 ml/min. The retention time under these conditions was 10.5 min.

Drug content and entrapment efficiency (EE) were calculated according to Eqs. (1) and (2), respectively. The mean value of three replicate determinations is reported.

$$\text{Drug content (\% w/w)}$$

$$= \frac{\text{mass of drug in nanoparticles} \times 100}{\text{mass of nanoparticles}}$$

$$\text{Entrapment efficiency (\% w/w)}$$

$$= \frac{\text{mass of drug in nanoparticles} \times 100}{\text{total mass of drug used in preparation of nanoparticles}}$$

PLA and D,L-PLGA 50/50 and 75/25 (lactic acid/glycolic acid) copolymers (Resomer 202H, RG 502 and 752 respectively) were purchased from Boehringer (Ingelheim, Germany). Polyvinyl alcohol (PVA) having a molecular weight of 27000 g/mol (Mowiol 4-98, Hoechst AG, Germany) was chosen as a stabilizing hydrocolloid. Acetone and acetonitrile were purchased from Merck (Germany). All other chemicals and solvents were of reagent grade and used as received.

Table: PLA/PLGA ratio and molecular weight of different polymers

| Type of polymer | PLA/PLGA ratio | MW [Da] |
|---|---|---|
| Resomer RG 502 | 50/50 | 14000 |
| Resomer RG 752 | 75/25 | 20000 |
| Resomer R 202 H | 100/0 | 17000 |

In the Examples an Encapsulator (Inotech IE-50 R, Inotech, Swiss) has been used as the vibrating nozzle device, which was originally used for formation of pellets and microcapsules.

The syringe pump which may be used in this embodiment was regulated with the syringe pump control system, that generates the selected electrical signal. On pumping speed display it could be indicated a number between 0 and 950, which is correlated to the syringe pump speed. Used value 353 corresponds to flow rate around 9 ml/min.

The preparation of NP was achieved by adjusting the nanoprecipitation technique, previously applied to the preparation of NP (Fessi et al. 1989).

EXAMPLE 1

*a) Preparation of nanoparticles (NP)*

550 mg of polylactic acid (PLA) / poly(lactic-co-glycolic acid) (PLGA) were dissolved in 15 ml of acetone and the solution was passed through a 500 $\mu$m nozzle of the encapsulator and allowed to drop into 200 ml of an aqueous polyvinyl alcohol solution (0.1-0.9 % w/w) which was stirred magnetically. The experiments were performed at a flow rate (stream velocity) of 8.8 ml/min. The vibration frequency used to break up the liquid jet was set at 482 Hz and the amplitude at 3.5. Afterwards, the organic solvent was evaporated under atmospheric pressure at room temperature and the aggregates were removed by filtration.

*b) Characterization of prepared nanoparticles*

Table 1

Effect of type of polymer and initial drug : polymer ratio on the mean particle diameter (d) and polydispersity index (PI) of nanoparticles. PVA concentration = 0.45 %, n = 3 (the experiment was done in triplicate).

Table 1

| Type of polymer | Initial ratio drug:polymer | d [nm] | PI |
|---|---|---|---|
| RG 502 | 1:5 | 224.0 +/- 10.6 | 0.15 +/- 0.01 |
| | 1:10 | 223.6 +/- 6.0 | 0.15 +/- 0.02 |
| | 1:20 | 225.6 +/- 1.6 | 0.11 +/- 0.05 |
| RG 752 | 1:10 | 269.4 +/- 3.4 | 0.20 +/- 0.01 |
| R 202 H | 1:10 | 242.4 +/- 5.6 | 0.24 +/- 0.01 |

Table 2

Effect of stabilizer concentration on the mean particle diameter (d) and the polydispersity index (PI) of nanoparticles. Polymer =Resomer RG 502, initial drug : polymer ratio = 1:10, n =3.

Table 2

| PVA (% w/V) | d [nm] | PI |
|---|---|---|
| 0.900 | 227.4 +/- 2.0 | 0.19 +/- 0.01 |
| 0.675 | 224.0 +/- 6.4 | 0.15 +/- 0.02 |
| 0.450 | 223.6 +/- 6.0 | 0.15 +/- 0.02 |
| 0.225 | 223.4 +/- 2.2 | 0.11 +/- 0.04 |
| 0.100 | 226.9 +/- 5.9 | 0.10 +/- 0.05 |

It can be seen from the above results that PLA and PLGA nanoparticles having a diameter below 250 nm can be readily prepared with good reproducibility by the present nanoprecipitation method using a vibrating nozzle device.

EXAMPLE 2

*Encapsulation of drugs*

The ability of the inventive method to incorporate drugs within the polymer nanoparticles is demonstrated using celecoxib (4-[5-(4-methylphenyl)-3-(trifluoromethyl)pyrazol-1-yl]benzenesulfonamide) as a drug. The substance celecoxib (27.5, 55 and 110 mg) was dissolved in a PLGA/acetone solution. Nanoparticles were fabricated using the procedures described in Example 1.

Table 3

Effect of type of polymer and initial drug : polymer ratio on entrapment efficiency (EE) and drug content of celecoxib in nanoparticles. PVA concentration = 0.45 %, n = 3.

Table 3

| Type of polymer | Initial ratio drug:polymer | % EE | drug content [%] |
|---|---|---|---|
| RG 502 | 1:5 | 83.9 +/- 1.7 | - |
| | 1:10 | 89.2 +/- 5.4 | 3.9 +/- 0.2 |
| | 1:20 | 98.6 +/- 1.4 | - |
| RG 752 | 1:10 | 70.8 +/- 7.2 | 3.4 +/- 0.6 |
| R 202 H | 1:10 | 71.0 +/- 6.9 | 3.4 +/- 0.3 |

Table 4

Effect of stabilizer concentration on entrapment efficiency (EE) and drug content of celecoxib in nanoparticles. Polymer =Resomer RG 502, initial drug : polymer ratio = 1:10, n =3.

Table 4

| PVA (% w/V) | % EE | drug content [%] |
|---|---|---|
| 0.900 | - | - |
| 0.675 | - | - |
| 0.450 | 89.2 +/- 5.4 | 3.9 +/- 0.2 |
| 0.225 | 84.0 +/- 3.0 | 5.2 +/- 0.5 |
| 0.100 | 82.2 +/- 4.2 | 5.9 +/- 0.1 |

Table 5

Effect of stabilizer (PVA) concentration and initial drug : polymer Resomer RG 502 ratio on drug content of celecoxib in nanoparticles. n =3.

Table

| % PVA in water phase | drug content [%] | |
|---|---|---|
| | drug/polymer = 1:5 | drug/polymer = 1:10 |
| 0.45 | 6.8 +/- 0.1 | 3.9 +/- 0.2 |
| 0.225 | 9.7 +/- 0.7 | 5.2 +/- 0.5 |
| 0.1 | 13.0 +/- 2.5 | 5.9 +/- 0.1 |

The highest drug content (13 %) in nanoparticles was obtained by using 0.1 % PVA solution as stabilizer and the initial ratio celecoxib : Resomer RG 502 = 1:5.

**Claims**

1. A method for preparing nanoparticles, comprising the steps of:

   dissolving a polymer and, optionally, at least one additional ingredient in an organic solvent,
   passing the solution through a vibrating nozzle, and
   dropping the solution through an electric field into an aqueous solution which is stirred,

   wherein the vibrating nozzle has a frequency of vibration in the range from 50 to 7000 Hz, and wherein the electric field is built up between said vibrating nozzle and an electrode and a voltage in the range from 800 to 1800 V is applied, such that nanoparticles are formed by the rapid diffusion of the solvent.

2. The method according to claim 1, further comprising the step of evaporating the solvent from the suspension under atmospheric pressure at room temperature or under reduced pressure.

3. The method according to claim 1 or 2, wherein the additional ingredient is a drug.

4. The method according to claim 3, wherein the drug is selected from the group consisting of pharmacologically active proteins, peptides, vaccines, celecoxib, budesonide, paclitaxel, camptothecin, 9-nitrocamptothecin, cisplatin, carboplatin, ciprofloxacin, doxorubicin, rolipram, simvastatin, methotrexate, indomethacin, probiprofen, ketoprofen, iroxicam, diclofenac, cyclosporine, etraconazole, rapamycin, nocodazole, colchicine, ketoconazole, tetracycline, minocycline, doxycycline, ofloxacin, octreotide, testosterone, progesterone, estradiol and estrogen.

5. The method according to any one of the preceding claims, wherein the aqueous solution comprises a stabilizer.

**6.** The method according to claim 5, wherein the stabilizer is selected from the group consisting of polyvinyl alcohol and poloxamers, or mixtures thereof.

**7.** The method according to claim 5 or 6, wherein the concentration of the stabilizer in the aqueous solution is $\leq 1$ % (w/w), preferably $\leq 0.1$ % (w/w).

**8.** The method according to any one of the preceding claims, wherein the organic solvent is selected from the group consisting of lower alcohol, a lower ketone, esters, acetonitrile or mixtures thereof, preferably acetone and/or ethanol.

**9.** The method according to any one of the preceding claims, wherein the polymer is selected from the group consisting of biodegradable polymers, preferably polylactide (PLA), copolymers of lactide and glycolide (PLGA), poly($\varepsilon$-caprolactone) (PCL) and non-biodegradable polymers, preferably copolymers of acrylic and methacrylic acid esters, cellulose acetate phthalate (CAP), cellulose acetate trimellitate (CAT), and ethylene vinyl acetate copolymer (EVAC), or mixtures thereof.

**10.** The method according to any of the preceding claims, wherein the polymer is a hydrophobic polymer.

**11.** The method according to any one of the preceding claims, wherein the concentration of the polymer in the organic solvent is in the range from 0.1 to 10 % w/w.

**12.** The method according to any one of the preceding claims, wherein the frequency of the vibration is in the range from 450 to 550 Hz.

**13.** The method according to any one of the preceding claims, wherein the amplitude of the vibration is in the range from 1 to 7, preferably in the range from 1 to 4.

**14.** The method according to any one of the preceding claims, wherein the flow rate of the solution passed through the nozzle is in the range from 8 to 10 ml/min.

**15.** The method according to any one of the preceding claims, wherein the voltage applied to the droplets is in the range from 800 to 1000 V.

**Patentansprüche**

**1.** Verfahren zum Herstellen von Nanopartikeln, welches die Schritte umfasst:

Lösen eines Polymers und wahlweise mindestens eines zusätzlichen Inhaltsstoffes in einem organischen Lösungsmittel,
Durchleiten der Lösung durch eine vibrierende Düse und Zutropfen der Lösung durch ein elektrisches Feld in eine wässrige Lösung, welche gerührt wird,
wobei die vibrierende Düse eine Vibrationsfrequenz im Bereich von 50 bis 7000 Hz aufweist, und wobei das elektrische Feld zwischen der besagten vibrierenden Düse und einer Elektrode angelegt wird und eine Spannung im Bereich von 800 bis 1800 V angelegt wird, so dass Nanopartikel durch rasche Diffusion des Lösungsmittels gebildet werden.

**2.** Verfahren gemäß Anspruch 1, welches weiterhin den Schritt der Verdampfung des Lösungsmittels aus der Suspension unter Atmosphärendruck bei Raumtemperatur oder unter reduziertem Druck umfasst.

**3.** Verfahren gemäß Anspruch 1 oder 2, wobei der zusätzliche Inhaltsstoff ein Wirkstoff ist.

**4.** Verfahren gemäß Anspruch 3, wobei der Wirkstoff aus der Gruppe ausgewählt wird, die aus pharmaklogisch aktiven Proteinen, Peptiden, Impfstoffen, Celecoxib, Budesonid, Paclitaxel, Camphothecin, 9- Nitrocamptothecin, Cisplatin, Carboplatin, Ciprofloxacin, Doxorubicin, Rolipram, Simvastatin, Methotrexat, Indomethacin, Probiprofen, Ketoprofen, Iroxicam, Diclofenac, Cyclosporin, Etraconazol, Rapamycin, Nocodazol, Colchinin, Ketoconazol, Tetracyclin, Minozyklin, Doxyzyklin, Ofloxacin, Octreotid, Testosteron, Progesteron, Estradiol und Östrogen besteht.

**5.** Verfahren gemäß irgendeinem der vorangehenden Ansprüche, wobei die wäßrige Lösung einen Stabilisator umfasst.

**6.** Verfahren gemäß Anspruch 5, wobei der Stabilisator aus der Gruppe bestehend aus Polyvinylalkohol und Poloxameren oder Mischungen davon ausgewählt wird.

**7.** Verfahren gemäß Anspruch 5 oder 6, wobei die Konzentration des Stabilisators in der wäßrigen Lösung ≤ 1 Gewichts-% beträgt, bevorzugt ≤ 0,1 Gewichts-%.

**8.** Verfahren gemäß irgendeinem der vorangehenden Ansprüche, wobei das organische Lösungsmittel aus der Gruppe bestehend aus niedrigen Alkoholen, eines niedrigen Keton, Estern, Acetonitril oder Mischungen davon ausgewählt wird, bevorzugt Aceton und/oder Ethanol.

**9.** Verfahren gemäß irgendeinem der vorangehenden Ansprüche, wobei das Polymer aus der Gruppe der biologisch abbaubaren Polymere, bevorzugt Polylaktid (PLA), Copolymeren von Laktid und Glykolid (PLGA), Poly($\varepsilon$-caprolakton) (PCL), und nicht biologisch abbaubaren Polymeren, vorzugsweise Copolymeren von Acryl- und Methacrylsäureestern, Celluloseacetat-phtalat (CAP), Celluloseacetat-Trimetallit (CAT) und Ethen-Vinlyacetat-Copolymer (EVAC), oder Mischungen davon ausgewählt wird.

**10.** Verfahren gemäß beliebiger der vorangehenden Ansprüche, wobei das Polymer ein hydrophobes Polymer ist.

**11.** Verfahren gemäß eines beliebigen der vorangehenden Ansprüche, wobei die Konzentration des Polymers im organischen Lösungsmittel im Bereich von 0,1 bis 10 Gewichts-% liegt.

**12.** Verfahren gemäß eines beliebigen der vorangehenden Ansprüche, wobei die Frequenz der Vibration im Bereich von 450 bis 550 Hz liegt.

**13.** Verfahren gemäß eines beliebigen der vorangehenden Ansprüche, wobei die Amplitude der Vibration im Bereich von 1 bis 7, vorzugsweise im Bereich von 1 bis 4 liegt.

**14.** Verfahren gemäß eines beliebigen der vorangehenden Ansprüche, wobei die Flussrate der Lösung, die durch die Düse geleitet wird, im Bereich von 8 bis 10 ml/min liegt.

**15.** Verfahren gemäß eines beliebigen der vorangehenden Ansprüche, wobei die Spannung, welche an die Tröpfchen angelegt wird, im Bereich von 800 bis 1000 V liegt.

**Revendications**

**1.** Procédé de préparation de nanoparticules, comprenant les étapes de :

dissolution d'un polymère et, facultativement, d'au moins un ingrédient supplémentaire dans un solvant organique,
passage de la solution à travers une buse vibrante, et
dépôt de la solution à travers un champ électrique dans une solution aqueuse qui est agitée,
dans lequel la buse vibrante a une fréquence de vibration dans la plage de 50 à 7 000 Hz, et dans lequel le champ électrique est créé entre ladite buse vibrante et une électrode, et une tension dans la plage de 800 à 1 800 V est appliquée, de telle sorte que les nanoparticules sont formées par la diffusion rapide du solvant.

**2.** Procédé selon la revendication 1, comprenant en outre l'étape d'évaporation du solvant depuis la suspension sous pression atmosphérique à température ambiante ou sous pression réduite.

**3.** Procédé selon la revendication 1 ou 2, dans lequel l'ingrédient supplémentaire est un médicament.

**4.** Procédé selon la revendication 3, dans lequel le médicament est choisi dans le groupe constitué des protéines pharmacologiquement actives, des peptides, des vaccins, du célécoxib, du budésonide, du paclitaxel, de la camptothécine, de la 9-nitrocamptothécine, du cisplatine, du carboplatine, de la ciprofloxacine, de la doxorubicine, du rolipram, de la simvastatine, du méthotrexate, de l'indométhacine, du probiprofène, du kétoprofène, de l'iroxicam, du diclofénac, de la cyclosporine, de l'étraconazole, de la rapamycine, du nocodazole, de la colchicine, du kétoconazole, de la tétracycline, de la minocycline, de la doxycycline, de l'ofloxacine, de l'octréotide, de la testostérone, de la progestérone, de l'oestradiol et de l'oestrogène.

**5.** Procédé selon l'une quelconque des revendications précédentes, dans lequel la solution aqueuse comprend un stabilisant.

**6.** Procédé selon la revendication 5, dans lequel le stabilisant est choisi dans le groupe constitué du polyalcool vinylique et des poloxamères, ou de leurs mélanges.

**7.** Procédé selon la revendication 5 ou 6, dans lequel la concentration du stabilisant dans la solution aqueuse est ≤ 1 % (p/p), de préférence ≤ 0,1 % (p/p).

**8.** Procédé selon l'une quelconque des revendications précédentes, dans lequel le solvant organique est choisi dans le groupe constitué d'un alcool inférieur, d'une cétone inférieure, des esters, de l'acétonitrile ou de leurs mélanges, de préférence l'acétone et/ou l'éthanol.

**9.** Procédé selon l'une quelconque des revendications précédentes, dans lequel le polymère est choisi dans le groupe constitué des polymères biodégradables, de préférence le polylactide (PLA), des copolymères de lactide et de glycolide (PLGA), de la poly(ε-caprolactone) (PCL) et des polymères non biodégradables, de préférence les copolymères d'esters d'acide acrylique et méthacrylique, de l'acétate phtalate de cellulose (CAP), de l'acétate trimellitate de cellulose (CAT) et du copolymère d'éthylène-acétate de vinyle (EVAC), ou de leurs mélanges.

**10.** Procédé selon l'une quelconque des revendications précédentes, dans lequel le polymère est un polymère hydrophobe.

**11.** Procédé selon l'une quelconque des revendications précédentes, dans lequel la concentration en le polymère dans le solvant organique est dans la plage de 0,1 à 10 % p/p.

**12.** Procédé selon l'une quelconque des revendications précédentes, dans lequel la fréquence de la vibration est dans la plage de 450 à 550 Hz.

**13.** Procédé selon l'une quelconque des revendications précédentes, dans lequel l'amplitude de la vibration est dans la plage de 1 à 7, de préférence dans la plage de 1 à 4.

**14.** Procédé selon l'une quelconque des revendications précédentes, dans lequel le débit de la solution passée à travers la buse est dans la plage de 8 à 10 mL/min.

**15.** Procédé selon l'une quelconque des revendications précédentes, dans lequel la tension appliquée aux gouttelettes est dans la plage de 800 à 1 000 V.

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5118528 A
- EP 1728814 A
- WO 2005072709 A2
- EP 0235603 A
- US 20040022939 A1
- US 6669961 B

### Non-patent literature cited in the description

- **H. FESSI ; F. PUISIEUX ; J.P. DEVISSAGUET ; N. AMMOURY ; S. BENITA.** Nanocapsule formation by interfacial polymer deposition following solvent displacement. *Int. J. Pharm.,* 1989, vol. 55, 25-28
- **PRAKOBVAITAYAKIT ; NIMMANNIT.** *AAPS PharmSciTech,* 2003, vol. 4 (4), 1-9
- **GOVENDER et al.** *Journal of Controlled Release,* 1999, vol. 57, 171-185
- **SAXENA et al.** *Int. J. Pharm,* 2004, vol. 278 (2), 293-301
- **CSABA et al.** *Journal of Biomaterials Science, Polymer edition,* 2004, vol. 15 (9), 1137-1151
- **BIOMACROMOLECULES.** *Biomacromolecules,* 2005, vol. 6, 271-278
- **AMELLER et al.** *European Journal of Pharmaceutical Sciences,* 2004, vol. 21, 361-370
- *Pharmaceutical Research,* 2003, vol. 20 (7), 1063-1070